# EUROPEAN PATENT APPLICATION

(11) **EP 4 748 391 A1**
(43) Date of publication of application: **27.05.2026**
(21) Application number: 24214220.6
(22) Date of filing: 20.11.2024
(51) Int. Cl.: A61K 39/09, C07K 14/00

(54) **N-TERMINAL REGION OF A GROUP B STREPTOCOCCUS SURFACE PROTEIN AS A CARRIER FOR THE CAPSULAR POLYSACCHARIDE**

(71) Applicant: MinervaX ApS, 2000 Frederiksberg (DK)
(72) Inventor: FISCHER, Per, 2000 FREDERIKSBERG (DK); JOHANSSON LINDBOM, Bengt, 224 57 LUND (SE)
(74) Representative: Öberg & Sjöberg AB

(57) **Abstract**

An immunogenic complex comprising i) an amino acid sequence of an N-terminal region of a group B Streptococcus surface protein selected from the group consisting of: a first amino acid sequence according to SEQ ID NO: 16, or an amino acid sequence having at least 98%, such as 99%, identity thereto; a second amino acid sequence according to SEQ ID NO: 18 or an amino acid sequence having at least 98%, such as 99%, identity thereto; and a third amino acid sequence according to SEQ ID NO: 20; and ii) a capsular polysaccharide, is provided. An immunogenic product, a vaccine, and the immunogenic complex, the immunogenic product or the vaccine for use in a method of preventing or treating a group B Streptococcus infection, are also provided.

## Description

### FIELD OF INVENTION

The present invention relates to the fields of microbiology and vaccine technology and concerns an immunogenic complex comprising an N-terminal region of a group B Streptococcus surface protein and a capsular polysaccharide. The invention further pertains to an immunogenic product comprising the immunogenic complex and an immunogenic fusion protein, a vaccine comprising the immunogenic complex and/or immunogenic product, and a method of preventing or treating a group B Streptococcus infection.

### BACKGROUND OF THE INVENTION

Group B Streptococcus (*Streptococcus agalactiae*) (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

GBS can also cause mastitis in cows, a bovine disease that is of considerable economical importance. Development of a vaccine against GBS infections is therefore of interest also in veterinary medicine.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease.

Group B streptococcal strains are divided into nine serotypes (Ia, Ib, and II-VIII) based on the structure of the polysaccharide capsule (Baker, J Inf Dis 1990. 161: 917). The four "classical" serotypes la, Ib, II, and III occur in roughly equal proportions among strains in the normal flora, but type III is the clinically most important serotype, in particular because it causes most cases of meningitis. Because the capsule is a known virulence factor, it has been studied in considerable detail, in particular in type III strains. Efforts have been made to develop a vaccine, in which the type III polysaccharide capsule would be an essential component.

EP 0 866 133 discloses a vaccine capable of protecting a recipient from infection caused by group B *Strepococcus.* The vaccine uses a combination of a polysaccharide and a fragment of the epsilon protein. It further discloses that epidemiological data suggest that the type-specific capsule plays an important role in the immunity to group B *Streptococcus* infections.

WO 9410317 describes the use of the alpha protein, a GBS surface protein, in the development of a conjugate vaccine. A drawback with this protein is that it usually is not expressed by type III strains, which are the cause of many serious GBS infections. Hence, a protective immunity against these strains will not be evoked by an alpha protein vaccine.

WO 2008127179 describes a fusion protein comprising at least one first N-terminal region fragment of a group B Streptococcus surface protein or analogue, homologue, derivative or immunologically related amino acid sequence or fragments thereof, which is fused to at least one second N-terminal region fragment of a group B Streptococcus surface protein or analogue, homologue, derivative or immunologically related amino acid sequence or fragments thereof, wherein the first and second at least one N-terminal region fragments of group B Streptococcus surface proteins derive from different group B Streptococcus strains, and wherein the fusion protein is capable of eliciting protective immunity against group B Streptococcus.

PCT/EP2016/080927 discloses immunogenic complexes comprising the N-terminal region of a group B Streptococcus surface protein, and a capsular polysaccharide. The immunogenic complex is capable of eliciting protective immunity against group B Streptococcus.

PCT/EP2022/058309 discloses immunogenic fusion proteins and their use for vaccination against group B Streptococcus infections.

Despite the advances in the progress towards a vaccine suitable for prevention of GBS disease, there is still a need for further methods and vaccines for prevention and treatment of GBS infections. Thus, there remains a need to explore vaccine strategies capable of eliciting protective immunity against a wide range of GBS stains.

Accordingly, it is a primary objective of the present invention to provide an immunogenic complex comprising an N-terminal region of a group B Streptococcus surface protein and a capsular polysaccharide which can be used in a vaccine capable of eliciting protective immunity against GBS infections.

It is a further objective of the present invention to provide a vaccine that elicits protective immunity against many clinically important GBS strains.

Another objective of the present invention is to provide a vaccine comprising a single, or a few, components that elicits protective immunity against GBS infections. A single or a few components has several advantages over a vaccine composed of numerous components, e.g. cost of production and safety.

The means of accomplishing each of the above objectives as well as others will become apparent from the description of the invention which follows hereafter.

### SUMMARY OF THE INVENTION

The present invention is based on realization, by the present inventors, that the non-immunodominant N-terminal regions of group B Streptococcus surface proteins, of which the use of the N-terminal regions of the surface proteins Rib and AlpC in the form of a fusion protein is disclosed in WO 2008127179, despite their non-immunodominancy can still be useful on their own and not only in the form of a fusion protein. The way to realise this usefulness is to employ these N-terminal regions as carriers for a capsular polysaccharide. As discussed above capsular polysaccharides have been used in vaccines, however, according to EP 0 866 133 the type-specific capsule plays a major role in the immunity, thus the width of protection against a range of different group B streptococcus strains is limited using capsular polysaccharides. By using an N-terminal region of a group B Streptococcus surface protein as a carrier for the capsular polysaccharide the immunogenicity and scope of protection will be increased.

In particular, the present inventors have now realized that the N-terminal carrier for the capsular polysaccharide may be further modified to further improve the resulting immunogenic complex.

Thus, a first aspect of the present invention relates to an immunogenic complex comprising:
i) an amino acid sequence of an N-terminal region of a group B Streptococcus surface protein selected from the group consisting of:
   a first amino acid sequence according to SEQ ID NO: 16, or an amino acid sequence having at least 98%, such as 99%, identity thereto;
   a second amino acid sequence according to SEQ ID NO: 18 or an amino acid sequence having at least 98%, such as 99%, identity thereto; and a third amino acid sequence according to SEQ ID NO: 20; and
ii) a capsular polysaccharide.

The immunogenic complex may be capable of eliciting protective immunity against group B Streptococcus.

Compared to the immunogenic complex of PCT/EP2016/080927, the immunogenic complex according to the first aspect as defined above uses a shorter amino acid sequence. Specifically, each of the first, second and third amino acid sequences and the corresponding SEQ ID Nos: 16, 18 and 20 have been modified in relation to the corresponding amino acid sequences of PCT/EP2016/080927 by removing one or more initial amino acids in the sequences. This may provide a number of advantages to the immunogenic complex such as decreasing the size of the immunogenic complex and making it easier to produce. Additionally, by removing one or more initial amino acids in the amino acid sequences the immunogenicity of the amino acid sequence part of the immunogenic complex may be increased, which leads to an increased immunogenicity of the whole immunogenic complex. These modifications may improve the efficacy of the immunogenic complex.

A second aspect of the present invention pertains to an immunogenic product comprising the immunogenic complex according to the first aspect of the present invention, wherein the immunogenic product further comprises an immunogenic fusion protein comprising:
a first amino acid sequence having at least 80% sequence identity with the amino acid sequence of the N-terminal region of a first group B Streptococcus surface protein, which is fused to
a second amino acid sequence having at least 80% sequence identity with the amino acid sequence of the N-terminal region of a second group B Streptococcus surface protein wherein each of the first and the second group B Streptococcus surface protein is selected from the group consisting of Rib protein, Alp1 protein, Alp2 protein, Alp3 protein, Alp4 protein and AlpC protein. The immunogenic fusion protein may be capable of eliciting protective immunity against group B Streptococcus.

Thus another advantage with the present invention is it also pertains to a immunogenic product which comprises the immunogenic complex according to the first aspect of the present invention combined with an immunogenic fusion protein, such as for example the Rib-AlpC-NN fusion protein of WO 2008127179, thus providing an immunogenic product capable of providing full coverage of protection against all clinically relevant Group B Streptococcus strains using only one immunogenic complex and one immunogenic fusion protein.

The third aspect of the present invention pertains to a vaccine comprising or consisting of a pharmaceutically acceptable vehicle, optionally an adjuvant, such as aluminium hydroxide, and a pharmaceutically effective amount of an immunogenic complex according to the first aspect of the present invention or a pharmaceutically effective amount of an immunogenic product according to the second aspect of the present invention, wherein the vaccine is capable of eliciting protective immunity against group B Streptococcus.

The corresponding fourth, fifth and sixth aspect of the present invention, respectively, pertain to the immunogenic complex according to the first aspect of the present invention, the immunogenic product according to the second aspect of the present invention, and/or the vaccine according to the third aspect of the present invention for use in a method of preventing or treating an infection caused by a group B Streptococcus;
a method of preventing or treating an infection caused by a group B Streptococcus comprising administering to a subject in need thereof a pharmaceutically effective amount of the immunogenic complex according to the first aspect of the present invention, the immunogenic product according to the second aspect of the present invention, and/or the vaccine according to the third aspect of the present invention; and
the use of an immunogenic complex according to the first aspect of the present invention, the immunogenic product according to the second aspect of the present invention for use in the preparation of a medicament for preventing or treating an infection caused by a group B Streptococcus.

### DETAILED DESCRIPTION OF THE INVENTION

In this specification, unless otherwise specified, "a" or "an" means "one or more".

Throughout the specification, any and all references are specifically incorporated into this patent application by reference.

In a first embodiment of the immunogenic complex according to the first aspect of the present invention the immunogenic complex comprises:
i) an amino acid sequence of an N-terminal region of a group B Streptococcus surface protein:
   selected from the group consisting of:
   a first amino acid sequence according to SEQ ID NO: 16, or an amino acid sequence having at least 98%, such as 99%, identity thereto;
   a second amino acid sequence according to SEQ ID NO: 18 or an amino acid sequence having at least 98%, such as 99%, identity thereto; and
   a third amino acid sequence according to SEQ ID NO: 20; and
ii) a capsular polysaccharide.

The immunogenic complex may be capable of eliciting protective immunity against group B Streptococcus.

The term "immunogenic" is intended to mean having the ability to elicit an immune response. The immunogenic complex of the invention is immunogenic and characterised by its ability to elicit a protective immune response against at least GBS expressing the surface protein of which the N-terminal region is comprised by, or GBS expressing the capsular polysaccharide.

In the complex the amino acid sequence works as a carrier for the capsular polysaccharide. Thus, the capsular polysaccharide may be covalently bound to the amino acid sequence.

According to the technology proposed herein, sequence identity is calculated in the following manner. By an amino or nucleic acid sequence having a sequence with at least, for example 95%, identity to a reference sequence, is intended that the sequence is identical to the reference sequence except that the sequence may include up to 5 amino or nucleic acid differences per each 100 amino or nucleic acids of the reference sequence. In other words, to obtain an amino or nucleic acid sequence having a sequence identity of at least 95%, up to 5% of the amino or nucleic acids in the reference sequence may be deleted or substituted with another amino or nucleic acid, or a number of amino or nucleic acids up to 5% of the total number of amino or nucleic acids in the reference sequence may be inserted into the reference sequence. If the two sequences to be compared are not of equal length, they must be aligned to best possible fit, and the sequence identity is calculated over the length of the reference sequence. These amino or nucleic acid changes of the reference sequence may occur at the amino and/or carboxy terminal positions for an amino acid sequence or at the 5' and/or 3' end for a nucleic acid sequence of a reference amino or nucleic acid sequence or anywhere between those terminal positions, interspersed either individually within the reference sequence or in one or more contiguous groups within the reference sequence. In the sequences of the present document, when such a sequence has or encodes an N-terminal methionine, this methionine or its coding sequence is optional and may optionally thus not to be taken into account when calculating sequence identities.

The term "N-terminal region" in relation to the present invention refers to an N-terminus region (N) of a protein. Examples of amino acid sequences of the N- terminal regions of the group B Streptococcus surface proteins are given in SEQ IDs NO: 2, 4, 8, 10, 14, 16, 18 and 20.

In particular, examples of N-terminal regions of group B Streptococcus proteins include the N-terminal region of the group B Streptococcus Rib, Alp1, Alp2, Alp3, Alp4 and AlpC protein, including peptides encoding native amino acid sequences of N-terminal regions of natural Rib, Alp1, Alp2, Alp3, Alp4 and AlpC protein.

Group B streptococcal strains, also referred herein as GBS, are well known and may be isolated from the blood of infected human beings. GBS is the most common cause of neonatal sepsis in the United States and is responsible for about 5000 cases per year. The denotation "Group B streptococcal" and "Group B streptococcus" derives from the fact that Streptococci have been divided into immunological groups based upon the presence of specific carbohydrate antigens on their cell surfaces. At present, groups A through O are recognized (Davis, B.D. et al., In: Microbiology, 3rd. Edition, page 609, (Harper & Row, 1980).

The capsular polysaccharide is preferably a bacterial polysaccharide, more preferably a group B Streptococcus polysaccharide.

The capsular polysaccharide may be serotype specific and selected from the group consisting of Group B Streptococcus serotypes la, Ib, II, III, IV, V, VI, VII, VIII, IX and X. By polysaccharide is meant any linear or branched polymer consisting of monosaccharide residues, usually linked by glycosidic linkages, and thus includes oligosaccharides. Preferably, the polysaccharide will contain between 2 and 50 monosaccharide unites, more preferably between 6 and 30 monosaccharide units.

The polysaccharide component may be based on or derived from polysaccharide components of the polysaccharide capsule from many Gram positive and Gram negative bacterial pathogens such as *H. influenzae, N. meningitidis* and *S*. *pneumoniae.* Other bacteria from which polysaccharide components may be conjugated to the carrier proteins of the present invention include *Staphylococcus aureus, Klebsiella, Pseudomonas, Salmonella typhi, Pseudomonas aeruginosa,* and *Shigella dysenteriae.* Polysaccharide components suitable for use according to this aspect of the present invention include the Hib oligosaccharide, lipopolysaccharide from *Pseudomonas aeruginosa* (Seid and Sadoff, 1981), lipopolysaccharides from Salmonella (Konadu *et al.,* 1996) and the O-specific polysaccharide from *Shigella dysenteriae* (Chu *et al.,* 1991). Other polysaccharide components suitable for use in accordance with the present invention will be well-known to those skilled in the art.

Fragments of bacterial capsular polysaccharide may be produced by any suitable method, such as by acid hydrolysis or ultrasonic irradiation (Szn *et al.,* 1986). Other methods of preparation of the polysaccharide components will be well known to those of skill in the art.

Preferably, as stated above, the polysaccharide is a capsular polysaccharide derived from group B Streptococcus, or their equivalents.

The capsular polysaccharide should preferably be coupled to the amino acid sequence by a covalent or non-covalent linkage. Any linkage known to the skilled person may be used to link a polysaccharide and an amino acid sequence according to the present invention. A particularly preferred method of coupling polysaccharide and the amino acid sequence is by reductive amination. Other methods include: activation of the polysaccharide with cyanogen bromide followed by reaction with adipic acid dihydrazide (spacer) and by conjugation to carboxide groups of carrier amino acid sequences or protein using soluble carbodiimides (Shneerson *et al.,* 1986); functionalisation of the carrier amino acid sequence or protein with adipic acid dihydrazide followed by coupling to cyanogen bromide activated polysaccharides (Dick *et al.,* 1989); chemical modification of both the carrier amino acid sequence and the polysaccharide followed by their coupling (Marburg *et al.,* 1986; Marburg *et al.,* 1987 and 1989).

The polysaccharide molecule may be coupled to the amino acid sequence by a spacer molecule, such as adipic acid. This spacer molecule can be used to facilitate the coupling of amino acid sequence to polysaccharide. A spacer may also be denoted a linker. Another non-limiting example of coupling of an amino acid sequence to a polysaccharide is non-covalent conjugation using biotin and a biotin-binding moiety. The polysaccharide may be biotinylated, and the amino acid sequence may comprise a biotin-binding moiety or the polysaccharide may comprise a biotin-binding moiety and the amino acid sequence may be biotinylated. The biotin-binding moiety may be rhizavidin. Another non-limiting example of non-covalent coupling is the use of a sialic acid-sialic acid binding moiety. The polysaccharide may comprise the sialic acid and the amino acid sequence may comprise a sialic acid-binding moiety or the polysaccharide may comprise the sialic acid-binding moiety and the amino acid sequence the sialic acid. The sialic acid-binding moiety may be SBD1, SBD2, SBD3, SBD4, NanH, NanH2, or VcNanH.

After the coupling reaction has been performed, the immunogenic complex or conjugate may be purified by diafiltration or other known methods to remove unreacted amino acid sequence or polysaccharide components.

If the polysaccharide is derived from a bacterial pathogen different from GBS, the conjugate may elicit immunity against two or more pathogens, e.g. multiple types of bacteria. This is a potentially important application of the immunogenic complex.

Multiple capsular polysaccharides may be coupled to the same amino acid sequence. Thus, the immunogenic complex may comprise multiple capsular polysaccharides each linked to the amino acid sequence by any of the techniques and/or linkers described above. Where the immunogenic complex comprises multiple capsular polysaccharides, the capsular polysaccharides may be identical or different. When the capsular polysaccharides are different, they may be derived from different bacteria, e.g. from different Group B Streptococcus serotypes.

The number of capsular polysaccharides in the immunogenic complex may thus be one or more such as 1, 2, 3, or more.

The term "protective immunity" in relation to the present invention refers to the ability of serum antibodies and/or cytotoxic T cell response induced during immunization to protect (partially or totally) against disease caused by an infectious agent, such as a group B Streptococcus. That is, a vertebrate immunized by the vaccines of the invention will experience limited growth and spread of group B Streptococcus. To determine whether protective immunity is induced by an immunogenic complex or vaccine, techniques well known for a person skilled in the art can be used. For example, to determine whether immunization with an immunogenic complex or vaccine according to the invention induces protective immunity against group B Streptococcus infection, immunized test animals can be challenged with group B Streptococcus and growth and spread of the group B Streptococcus is measured.

The N-terminal regions of the group B Streptococcus surface proteins used in the different aspects of the present invention are from the group consisting of Rib protein, AlpC protein. Alp2 protein, and Alp3 protein.

This is advantageous as these proteins are expressed by clinically relevant group B Streptococcus serotypes/strains.

The group B Streptococcus Rib protein, also referred to in this specification as Rib and Rib protein, is a surface protein known in the art, and for example described in WO 9421685. The denotation "Rib" refers to: Resistance to proteases, immunity, and group B. The Rib protein was first isolated from a group B streptococcal strain of serotype III as a distinct 95 kDa protein. Protein Rib is expressed by almost all group B streptococcal strains of the clinically important serotype III, which cause most cases of meningitis, and by some strains of other serotypes such as II. Moreover, Rib is expressed by all strains of a hypervirulent clone of type III. A method has been devised to purify protein Rib and it has been demonstrated that antibodies to this protein protect against lethal infection with strains expressing protein Rib (for further details, such as DNA and protein sequences see WO 9421685). Nucleic acid sequences and amino acid sequences for the N-terminal region of Rib are given in SEQ ID Nos: 1, 2, 15 and 16.

The nucleic acid sequence and the amino acid sequence for the N-terminal region of Alp1 are given in SEQ ID Nos: 7 and 8.

The Alp2 protein is another alpha-protein-like-protein first identified in a serotype V strain (Lachenauer, C. S., R. Creti, J. L. Michel, and L. C. Madoff. 2000. Mosa- icism in the alpha-like protein genes of group B streptococci. Proc. Natl. Acad. Sci. USA 97:9630-9635.). Like the other members of the family, the Alp2 protein has an N-terminal domain and several repeated domains towards the C-terminus. Subsequently that protein has been found also in other GBS isolates such as serotypes Ia and III (Lindahl et al. Surface Proteins of Streptococcus agalactiae and Related Proteins in Other Bacterial Pathogens, CLINICAL MICROBIOLOGY REVIEWS, Jan. 2005, p. 102-127). Nucleic acid sequences and amino acid sequences for the N-terminal region of Alp2 are given in SEQ ID Nos: 9, 10, 19 and 20.

The Alp3 protein is yet another alpha-protein-like-protein, also know as R28. It is very similar to the R28 protein also found in S. pyrogenes. (Lachenauer, C. S., R. Creti, J. L. Michel, and L. C. Madoff. 2000. Mosa- icism in the alpha-like protein genes of group B streptococci. Proc. Natl. Acad. Sci. USA 97:9630-9635 and Lindahl et al. Surface Proteins of Streptococcus agalactiae and Related Proteins in Other Bacterial Pathogens, CLINICAL MICROBIOLOGY REVIEWS, Jan. 2005, p. 102-127). The structure is more complex than the other Alpha-protein-like-proteins, but it retains an N-terminal domain which is identical to that of Alp2, and C-terminal repeat regions very similar to Rib. Nucleic acid sequences and amino acid sequences for the N-terminal region of Alp3 are the same as for Alp2 and are given in SEQ ID Nos: 9, 10, 19 and 20.

The nucleic acid sequence and the amino acid sequence for the N-terminal region of Alp4 are given in SEQ ID Nos 13 and 14.

The group B Streptococcus AlpC protein, also known as alpha protein, is a group B Streptococcus surface protein known in the art. WO 9410317 describes a conjugate vaccine composition comprising the alpha protein. The native group B Streptococcus AlpC precursor protein as described in WO 9410317 has a molecular weight of 108 kDa. Cleavage of the putative signal sequence of 41 amino acids yields a mature protein of 104 kDa. (Note, however, that the signal sequence was subsequently shown to have a length of 56 amino acid residues: Stålhammar-Carlemalm et al., J Exp Med 177,1593; 1993).

The 20 kDa N-terminal region of the AlpC antigen shows no homology to previously described protein sequences and is followed by a series of nine tandem repeating units that make up 74% of the mature protein. Each repeating unit (denoted herein as "R") is identical and consists of 82 amino acids with a molecular mass of about 8500 Daltons, which is encoded by 246 nucleotides. The C-terminal region of the AlpC antigen contains a cell wall anchor domain motif present in a number of Gram-positive surface proteins. Nucleic acid sequences and amino acid sequences for the N-terminal region of AlpC are given in SEQ ID Nos: 3, 4, 17 and 18.

Each of the Rib, Alp1, and AlpC proteins of GBS includes a unique N-terminal region (N) and a long repeat (R) region. The proteins expressed by the GBS strains BM110 and A909 have 12 and 9 repeats, respectively. The wall anchoring regions are located at the C-terminal ends.

The N-terminal regions of Alp2 and Alp3 are identical.

The tandem repeats in Rib and alpha are identical within each protein, but not between the proteins, and vary in number between isolates. Except for this variation, the sequences of Rib and alpha are stable among strains. The two proteins show little or no antigenic cross-reactivity.

In one embodiment of the immunogenic complex according to the first aspect of the present invention, the first amino acid sequence consists of SEQ ID NO: 16 or an amino acid sequence having at least 98% or at least 99% identity thereto. The first amino acid sequence may have a length of from 172 amino acids to 178 amino acids.

In one embodiment of the immunogenic complex according to the first aspect of the present invention, the second amino acid sequence consists of SEQ ID NO: 18 or an amino acid sequence having at least 98% or at least 99% identity thereto. The second amino acid sequence may have a length of from 167 amino acids to 173 amino acids.

In one embodiment of the immunogenic complex according to the first aspect of the present invention, the third amino acid sequence consists of SEQ ID NO: 20.

In one embodiment of the immunogenic complex according to the first aspect of the present invention, the amino acid sequence is either said first amino acid sequence, said second or said third amino acid sequence. The immunogenic complex may thus comprise only one of the first, second and third amino acid sequences together with the capsular polysaccharide.

In one embodiment of the immunogenic complex according to the first aspect of the present invention, the immunogenic complex comprises two or all three of said first amino acid sequence, said second amino acid sequence and said third amino acid sequence.

In one embodiment of the immunogenic complex according to the first aspect of the present invention the immunogenic complex further comprises a further amino acid sequence having at least 80% sequence identity, preferably at least 90% sequence identity, more preferably at least 95% sequence identity, with the amino acid sequence of the N-terminal region of a further group B Streptococcus surface protein.

This is advantageous as it provides for an immunogenic fusion protein capable of eliciting protective immunity against a larger number of group B Streptococcus strains. The further amino acid sequence may be fused to the first, second or third amino acid sequence to form a fusion protein. Further, two of the first, second and third amino acid sequences may be fused with each other to form a fusion protein.

In a further embodiment of the immunogenic complex according to the first aspect of the present invention the further N-terminal region of a group B Streptococcus surface protein is selected from the group consisting of Rib protein, Alp1 protein, Alp2 protein, Alp3 protein, Alp 4 protein and AlpC protein.

More preferably the further N-terminal region of a group B Streptococcus surface protein is selected from the group consisting of Alp1 protein, Alp2 protein, Alp3 protein, and Alp 4 protein, such as the group consisting of Alp2 protein, Alp3 protein, and Alp 4 protein.

Where the immunogenic complex comprises multiple capsular polysaccharides each of the capsular polysaccharides may be carried, by being covalently attached or attached via a linker or otherwise attached as described above, to the first, second, third or further amino acid sequence. Thus, where the immunogenic complex comprises two capsular polysaccharides and two amino acid sequences, one of the capsular polysaccharides may be attached to one of the amino acid sequences and the other of the capsular polysaccharides may attached to the other one of the amino acid sequences.

In one embodiment of the immunogenic complex according to the first aspect of the present invention the first, second and/or third amino acid sequence and the further group B Streptococcus surface proteins are derived from different group B Streptococcus strains. Thus, the further group B Streptococcus surface protein is preferably not Rib when said immunogenic complex comprises said first amino acid sequence, is not AlpC when said immunogenic complex comprises said second amino acid sequence and/or is not Alp2 when said immunogenic complex comprises said third amino acid sequence. This will imply variability in the sequence of the N-terminal region fragments but would not alter the biological properties and their functional ability to elicit protective immunity. This is advantageous as it increases the number of group B Streptococcus strains which the immunogenic complex according to the first aspect of the present invention provides protection against.

In one preferred embodiment of the immunogenic complex according to the first aspect of the present invention only one of the first, second and third amino acids sequences, preferably the first or second, is present in the immunogenic complex.

In a further embodiment of the immunogenic complex according to the first aspect of the present invention the amino acid sequence and the capsular polysaccharide, and optionally also the further amino acid sequence of the N-terminal of a group B Streptococcus surface protein, are derived from different group B Streptococcus serotypes.

This is advantageous as it increases the number of group B Streptococcus serotypes which the immunogenic complex according to the first aspect of the present invention provides protection against.

In a further embodiment of the immunogenic complex according to the first aspect of the present invention the further amino acid sequence has at least 80%, such as at least 85%, such as at least 90%, such as 95, 96, 97, 98 or 99 % sequence identity with one of the amino acid sequences SEQ IDs 2, 4, 8, 10, 14, 16, 18 and 20.

In the preferred embodiment of the immunogenic complex according to the first aspect of the present invention the amino acid sequence, and optionally also the further amino acid sequence, is conjugated to the capsular polysaccharide.

Conjugation (coupling) encompasses covalently attaching, either directly or via a linker structure or chain and methods non-covalently conjugating an amino acid sequence to a polysaccharide as described above. Where the immunogenic complex comprises multiple capsular polysaccharides, each capsular polysaccharide may alternatively be conjugated to a single one of the amino acid sequences.

In one embodiment of the immunogenic complex according to the first aspect of the present invention the amino acid sequence, and optionally also the further amino acid sequence, is modified by glycosylation, amidation, carboxylation or phosphorylation, or by being conjugated to an RSV antigen.

RSV antigens are described below with reference to the third aspect of the present invention.

This is advantageous as such polypeptides, i.e. amino acid sequences, may have enhanced immunogenicity. Such polypeptides may result when the native forms of the polypeptides or fragments thereof are modified or subjected to treatments to enhance their immunogenic character in the intended recipient. Numerous techniques are available and well known to those of skill in the art which may be used, without undue experimentation, to substantially increase the immunogenicity of the polypeptides herein disclosed. For example, the polypeptides may be modified by coupling to dinitrophenol groups or arsanilic acid, or by denaturation with heat and/or SDS. For a review of some general considerations in coupling strategies, see Antibodies, A Laboratory Manual, Cold Spring Harbor Laboratory, ed. E. Harlow and D. Lane (1988). Useful immunogenic carriers are well known in the art. Examples of such carriers are keyhole limpet hemocyanin (KLH); albumins such as bovine serum albumin (BSA) and ovalbumin, PPD (purified protein derivative of tuberculin); red blood cells; tetanus toxoid; cholera toxoid; agarose beads; activated carbon; or bentonite.

A second aspect of the present invention concerns an immunogenic product comprising the immunogenic complex according to the first aspect of the present invention, wherein the immunogenic product further comprises an immunogenic fusion protein comprising:
a first amino acid sequence having at least 80% sequence identity with the amino acid sequence of the N-terminal region of a first group B Streptococcus surface protein, which is fused to
a second amino acid sequence having at least 80% sequence identity with the amino acid sequence of the N-terminal region of a second group B Streptococcus surface protein wherein each of the first and the second group B Streptococcus surface protein is selected from the group consisting of Rib protein, Alp1 protein, Alp2 protein, Alp3 protein, Alp4 protein and AlpC protein. The immunogenic fusion protein may be capable of eliciting protective immunity against group B Streptococcus.

This is advantageous as it provides an immunogenic product capable of providing full coverage of protection against all clinically relevant Group B Streptococcus strains using only one immunogenic complex and one immunogenic fusion protein.

In a preferred embodiment of the immunogenic product according to the second aspect of the present invention the first amino acid sequence of the fusion protein has at least 80%, such as at least 85%, such as at least 90%, such as 95, 96, 97, 98 or 99 % sequence identity with one of the amino acid sequences SEQ IDs 2, 4, 8, 10, 14, 16, 18 and 20, and wherein the second amino acid sequence of the fusion protein has at least 80%, such as at least 85%, such as at least 90%, such as 95, 96, 97, 98 or 99 % sequence identity with one of the amino acid sequences SEQ IDs 2, 4, 8, 10, 14, 16, 18 and 20,
or alternatively
the immunogenic fusion protein comprises an amino acid sequence having at least 80%, such as at least 85%, such as at least 90%, such as 95, 96, 97, 98 or 99 % sequence identity with any one of the amino acid sequences SEQ ID NO:6, 12 and 22. The corresponding nucleic acid sequences for SEQ ID Nos 6, 12 and 22 are given in SEQ ID Nos 5, 11 and 21, respectively.

For the purpose of the present invention the term "fusion protein" refers to an assembly of two or more proteins or or regions of proteins, comprising for example an N-terminal region of a group B Streptococcus Alp1 protein and an N-terminal region of a group B Streptococcus Alp2 protein. For example, there might be one N-terminal region of the A!p1- and one N-terminal region of the Alp2, or 2, 3, 4 or 5 N-terminal region fragments of the A!p1- and the Alp2-proteins, wherein the numbers of N-terminal regions from the two proteins need not be equal.

The combination of polypeptides to provide a fusion protein can be accomplished by coupling or conjugation, either directly or through an intermediate structure, or by molecular biological fusion, i.e. through the combination of recombinant nucleic acid molecules which comprise fragments of nucleic acid capable of encoding each of the two, such that a single continuous expression product is finally produced.

For the purpose of the present invention the term "protein" refers to a molecular chain of amino acids. A protein is not of a specific length and can, if required, be modified in vivo or in vitro, by, for example, glycosylation, amidation, carboxylation or phosphorylation. Inter alia, amino acid sequences, peptides, oligopeptides and polypeptides are included within the definition. The protein or peptide can be of natural or synthetic origin. In this context a fusion protein is intended to mean two or more polypeptides covalently linked to each other either directly or indirectly by several means such as those mentioned above. The term "fused" means to create a fusion protein as mentioned above.

In addition to, or as replacement for, the immunogenic fusion protein the immunogenic product may comprise one or more further immunogenic complexes according to the first aspect of the present invention. Thus, the immunogenic product according to the second aspect of the present invention may for example comprise only two immunogenic complexes according to the first aspect of the present invention, only one immunogenic complex and one immunogenic fusion protein as discussed above, two or more immunogenic complexes according to the first aspect of the present invention and one immunogenic fusion protein as discussed above, or two or more immunogenic fusion complexes according to the first aspect of the present invention and two or more immunogenic fusion proteins as discussed above. In each case the amino acid sequence and capsular polysaccharide of the immunogenic complex or complexes may be derived from the same or different serotypes/strains, and the first and second amino acid sequences of the immunogenic fusion protein or fusion proteins may correspond to the N-terminal regions of the same or different group B Streptococcus surface proteins. Preferably, in order to obtain a wide scope of protection, each capsular polysaccharide and amino acid sequence, be it in an immunogenic complex or in an immunogenic fusion protein, is derived from different serotypes/strains, and for the amino acid sequences, from different group B Streptococcus surface proteins.

Another embodiment of the immunogenic product comprises a first immunogenic complex wherein the amino acid sequence of an N-terminal region of a group B Streptococcus surface protein is SEQ ID NO: 16, a second immunogenic complex wherein the amino acid sequence of an N-terminal region of a group B Streptococcus surface protein is SEQ ID NO: 20, and optionally a third immunogenic complex wherein the amino acid sequence of an N-terminal region of a group B Streptococcus surface protein is SEQ ID NO: 18.

In another embodiment of the immunogenic product according to the second aspect of the present invention the further group B Streptococcus surface protein is selected from the group consisting of Alp1 protein, Alp2 protein, Alp3 protein, and Alp 4 protein.

A third aspect of the present invention concerns a vaccine comprising a pharmaceutically acceptable vehicle, optionally an adjuvant, and a pharmaceutically effective amount of an immunogenic complex according to the first aspect of the present invention and/or a pharmaceutically effective amount of an immunogenic product according to the second aspect of the present invention, wherein the vaccine is capable of eliciting protective immunity against group B Streptococcus.

The term "pharmaceutical acceptable vehicle" is intended to mean any suitable acceptable excipient, adjuvants, carrier, diluent commonly used in pharmaceutical formulations.

The vaccine may be a vaccine composition.

The vaccine may, in addition to the immunological complex and/or the immunological product, comprise other pharmacologically acceptable ingredients such as salts, buffers, immunoactive components, adjuvants (e.g. AIOH), wetting agents, emulsifying and suspending agents, or sweetening, flavouring, perfuming agents, or other substances which are desirable for improving the efficacy of the composition. A composition is said to be "pharmacologically acceptable" if its administration can be tolerated by a recipient individual.

A multivalent vaccine may also be prepared by combining the immunogenic complex or the immunogenic product with other components, including but not limited to other fusion proteins as described above, diphtheria toxoid or tetanus toxoid, or polysaccharides, using techniques known in the art. The vaccine may further comprise further antigens such as RSV antigens or *E. coli* antigens.

Methods for the preparation and formulation of vaccines and vaccine compositions are well known to those skilled in the art. The choice of ingredients will for instance vary depending on the administration route of the composition. For example, compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Liquid dosage forms for oral administration may generally comprise a liposome solution containing the liquid dosage form. Suitable forms for suspending liposomes include emulsions, suspensions, solutions, syrups, and elixirs containing inert diluents commonly used in the art, such as purified water.

In a further embodiment of the third aspect of the present invention the vaccine may comprise an additional immunoactive component. The additional immunoactive component may be an antigen, an immune enhancing substance, and/or a vaccine; either of these may comprise an adjuvant.

Adjuvants are substances that can be used to specifically augment a specific immune response. Normally, the adjuvant and the composition are mixed prior to presentation to the immune system, or presented separately, but into the same site of the animal or human being immunized. Adjuvants can be loosely divided into several groups based upon their composition. These groups include oil adjuvants (for example, Freund's complete and incomplete), mineral salts for example, AIK (SO4)2, AINa (SO4)2, AINH4 (SO4), AIOH, silica, kaolin, and carbon), polynucleotides (for example, poly IC and poly AU acids), and certain natural substances (for example, wax D from Mycobacterium tuberculosis, as well as substances found in Corynebacterium parvum, or Bordetella pertussis, and members of the genus Brucella. Among those substances particularly useful as adjuvants are saponins such as, for example, Quil A. Examples of materials suitable for use in vaccine compositions are provided in Remington's Pharmaceutical Sciences (Osol, A, Ed, Mack Publishing Co, Easton, PA, pp. 1324-1341 (1980).

The vaccine according to the third aspect of the present invention may be administrated parenterally, intramuscularly, intravenously, intraperitoneally, intradermally, mucosally, submucosally, topically or subcutaneously.

The vaccine according to the third aspect may further comprise more than one immunogenic complex according to the first aspect of the present invention. Thus, the vaccine may for example comprise a first immunogenic complex where the amino acid sequence of an N-terminal region of a group B Streptococcus surface protein is SEQ ID NO: 16 or has at least 98% sequence identity thereto, a second immunogenic complex where the amino acid sequence of an N-terminal region of a group B Streptococcus surface protein is SEQ ID NO: 20 or has at least 98% sequence identity thereto, and optionally a third immunogenic complex where the amino acid sequence of an N-terminal region of a group B Streptococcus surface protein is SEQ ID NO: 18. The capsular polysaccharides of the first, second, and optionally, third immunogenic complexes may be derived from the same serotype/strain, however it is preferred that the capsular polysaccharides are derived from different serotypes/strains.

Further the vaccine may comprise both an immunogenic complex according to the first aspect of the present invention and an immunogenic product according to the second aspect of the present invention.

The vaccine preferably comprises a pharmaceutically effective amount of an immunogenic product in which the amino acid sequence of an N-terminal region of a Group B Streptococcus surface protein is SEQ ID NO: 20, or in which there is only one of the first, second and third amino acid sequences of an N-terminal region of a group B Streptococcus surface protein.

Preferable the vaccine comprises aluminium hydroxide as an adjuvant.

Thus, in one embodiment, the vaccine consists of a pharmaceutically acceptable vehicle, aluminium hydroxide, and an immunogenic product in which there is only one of the first, second and third amino acid sequences of an N-terminal region of a group B Streptococcus surface protein.

The corresponding fourth, fifth and sixth aspect of the present invention, respectively, pertain to the immunogenic complex according to the first aspect of the present invention, the immunogenic product according to the second aspect of the present invention, and/or the vaccine according to the third aspect of the present invention for use in a method of preventing or treating an infection caused by a group B Streptococcus;
a method of preventing or treating an infection caused by a group B Streptococcus comprising administering to a subject in need thereof a pharmaceutically effective amount of the immunogenic complex according to the first aspect of the present invention, the immunogenic product according to the second aspect of the present invention, and/or the vaccine according to the third aspect of the present invention; and
and the use of an immunogenic complex according to the first aspect of the present invention, the immunogenic product according to the second aspect of the present invention for use in the preparation of a medicament, such as a vaccine, for preventing or treating an infection caused by a group B Streptococcus.

The immunogenic complex according to the first aspect of the present invention, the immunogenic product according to the second aspect of the present invention, and/or the vaccine according to the third aspect of the present invention may be administered in a pharmaceutically effective amount to an individual.

The term "pharmaceutically effective amount" in relation to the present invention refers to that amount which provides a therapeutic effect for a given condition and administration regimen. This is a predetermined quantity of active material calculated to produce a desired therapeutic effect in association with the required additives and diluents; i.e., a carrier, or administration vehicle. Further, it is intended to mean an amount sufficient to reduce and most preferably prevent a clinically significant deficit in the activity and response of the host. Alternatively, a pharmaceutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluents; i.e., carrier, or additive. Further, the dosage to be administered will vary depending on the active principle or principles to be used, the age, weight etc of the individual to be treated.

The terms "preventing or treating" in its various grammatical forms in relation to the present invention refer to preventing, curing, reversing, attenuating, alleviating, ameliorating, inhibiting, minimizing, suppressing, or halting (1) the deleterious effects of a disorder associated with group B Streptococcus infection, (2) disorder progression, or (3) disorder causative agent (group B Streptococcus). Further, the terms "preventing or treating" are contemplated to include the creation of total or partial immunity of the individual to group B Streptococcus infection.

Maternal immunoprophylaxis with a vaccine, for protecting against infection to group B Streptococcus both in the mother and in the young infant, has long been proposed as a potential route.

Thus, some embodiments of the corresponding forurth and fifth aspects of the present invention comprise administering to a human female a pharmaceutically effective amount of an immunogenic complex, immunogenic product, or vaccine as described herein capable of conferring immunity to the infection to an unborn offspring of the human female.

According to these embodiments, the vaccine is administered to a non-pregnant female or to a pregnant female, under conditions of time and amount sufficient to cause the production of antibodies which serve to protect both the female and a fetus or newborn (via passive transfer of antibodies across the placenta).

A further aspect of the present invention concerns a method for preventing or treating an infection caused by a group B Streptococcus which comprises administering to an individual in need thereof a pharmaceutically effective amount of antibodies elicited from the exposure of a second individual to an immunogenic complex, immunogenic product and/or a vaccine according to the first, second and/or third aspects of the present invention.

According to this aspect, resistance to group B Streptococcus is conferred to the individual by passive immunization, i.e., the immunogenic complex, immunogenic product and/or vaccine is provided to a host (i.e. a human or mammal) volunteer, and the elicited antisera is recovered and directly provided to a recipient suspected of having an infection caused by a group B Streptococcus. It is contemplated that such antisera could be administered to a pregnant female (at or prior to parturition), under conditions of time and amount sufficient so that the antisera would serve to protect either the fetus or newborn (via passive incorporation of the antibodies across the placenta).

The vaccine or antisera of the present invention may, thus, be provided either prior to the onset of infection (so as to prevent or attenuate an anticipated infection) or after the initiation of an actual infection.

The vaccine may be administered to a subject such as humans or animals, including mammals and birds, such as rodents (mouse, rat, guinea pig, or rabbit); birds (turkey, hen or chicken); other farm animals (cow, horse, pig or piglet); pets (dog, cat and other pets); and humans. While many animals may be treated with the vaccine of the invention, a preferred individual for treatment is a human or commercially valuable animal and livestock such as fish, e.g. Tilapia, and camels.

The vaccine can be administered to an individual according to methods known in the art. Such methods comprise application e.g. parenterally, such as through all routes of injection into or through the skin: e.g. intramuscular, intravenous, intraperitoneal, intradermal, mucosal, submucosal, or subcutaneous. Also, they may be applied by topical application as a drop, spray, gel or ointment to the mucosal epithelium of the eye, nose, mouth, anus, or vagina, or onto the epidermis of the outer skin at any part of the body. Other possible routes of application are by spray, aerosol, or powder application through inhalation via the respiratory tract. In this last case the particle size that is used will determine how deep the particles will penetrate into the respiratory tract. Alternatively, application can be via the alimentary route, by combining with the food, feed or drinking water e.g. as a powder, a liquid, or tablet, or by administration directly into the mouth as a: liquid, a gel, a tablet, or a capsule, or to the anus as a suppository. The vaccine may also be administrated in the form of a DNA vaccine.

Many different techniques exist for the timing of the immunizations. It is possible to use the immunogenic complex, immunogenic product and/or vaccine more than once to increase the levels and diversities of expression of the immunoglobulin repertoire expressed by the immunized animal. Typically, if multiple immunizations are given, they will be given one to two months apart.

In the immunogenic product the preferred human dose of the immunogenic fusion protein in the presence of Alhydrogel is within the range of 1 to 250 µg, preferably 10 to 150 µg, preferably 25 to 100 µg or 40 to 80 µg. In the absence of Alhydrogel, the preferred human doses of the immunogenic fusion protein would be 10 to 100 µg, preferably 50 to 500 µg, or preferably 100 to 250 µg.

Generally, the dosage may consist of an initial injection, most probably with adjuvant, followed most probably by one or maybe more booster injections. Preferably, booster injections may be administered at about 1 and 6 months after the initial injection.

**Table 1: Table of sequences. N-terminal methionines are optional.**

| SEQ ID NO | Sequence | Denotation |
|---|---|---|
| 1 | gggcccctgg gatccgctga agtaatttca ggaagtgctg ttacgttaaa cacaaatatg actaaaaatg ttcagaatgg tagagcatat atagatttat atgatgtgaa aaatgggaaa atagatccat tacaattaat tacgttaaat tcacctgatt taaaagctca gtatgtcatt aggcaaggcg gcaattattt cacacaacct tctgaattga ctactgttgg tgcagctagt attaattata cagtattgaa gacagatgga agtcctcata cgaagcctga tggacaagtg gatattataa acgtttcatt gactatttac aattcttcag ctttgagaga taaaatagat gaagttaaaa agaaagcgga agaccctaaa tgggacgagg gaagtcgcga taaagttttg ataagtttag atgatatcaa aacagatatt gataataatc ctaagacgca atcagacatt gccaataaaa taactgaagt tactaattta gaaaaaatac tagtacctcg aatccca | Nucleic acid sequence of SEQ ID NO: 2 |
| 2 | GPLGSAEVISGSAVTLNTNMTKNVQNGRAYIDLYDVKNGKID PLQLITLNSPDLKAQYVIRQGGNYFTQPSELTTVGAASINYTV LKTDGSPHTKPDGQVDIINVSLTIYNSSALRDKIDEVKKKAED PKWDEGSRDKVLISLDDIKTDIDNNPKTQSDIANKITEVTNLEK ILVPRIP | Rib N-terminal region |
| 3 | gggcccctgg gatcctctac aattccaggg agtgcagcga ccttaaatac aagcatcact aaaaatatac aaaacggaaa tgcttacata gatttatatg atgtaaaatt aggtaaaata gatccattac aattaattgt tttagaacaa ggttttacag caaagtatgt ttttagacaa ggtactaaat actatgggga tgtttctcag ttgcagagta caggaagggc tagtcttacc tataatatat ttggtgaaga tggactacca catgtaaaga ctgatggaca aattgatata | Nucleic acid sequence of SEQ ID NO: 4 |
| | gttagtgttg ctttaactat ttatgattca acaaccttga gggataagat tgaagaagtt agaacgaatg caaacgatcc taagtggacg gaagaaagtc gtactgaggt tttaacagga ttagatacaa ttaagacaga tattgataat aatcctaaga cgcaaacaga tattgatagt aaaattgttg aggttaatga attagagaaa ttgttagtat tgtca | |
| 4 | GPLGSSTIPGSAATLNTSITKNIQNGNAYIDLYDVKLGKIDPLQ LIVLEQGFTAKYVFRQGTKYYGDVSQLQSTGRASLTYNIFGE DGLPHVKTDGQIDIVSVALTIYDSTTLRDKIEEVRTNANDPKW TEESRTEVLTGLDTIKTDIDNNPKTQTDIDSKIVEVNELEKLLV LS | AlpC N-terminal region |
| 5 | gggcccctgg gatccgctga agtaatttca ggaagtgctg ttacgttaaa cacaaatatg actaaaaatg ttcagaatgg tagagcatat atagatttat atgatgtgaa aaatgggaaa atagatccat tacaattaat tacgttaaat tcacctgatt taaaagctca gtatgtcatt aggcaaggcg gcaattattt cacacaacct tctgaattga ctactgttgg tgcagctagt attaattata cagtattgaa gacagatgga agtcctcata cgaagcctga tggacaagtg gatattataa acgtttcatt gactatttac aattcttcag ctttgagaga taaaatagat gaagttaaaa agaaagcgga agaccctaaa tgggacgagg gaagtcgcga taaagttttg ataagtttag atgatatcaa aacagatatt gataataatc ctaagacgca atcagacatt gccaataaaa taactgaagt tactaattta gaaaaaatac tagtacctcg aatcccagaa ttctctacaa ttccagggag tgcagcgacc ttaaatacaa gcatcactaa aaatatacaa aacggaaatg cttacataga tttatatgat gtaaaattag gtaaaataga tccattacaa ttaattgttt tagaacaagg ttttacagca aagtatgttt ttagacaagg tactaaatac tatggggatg tttctcagtt gcagagtaca ggaagggcta gtcttaccta taatatattt ggtgaagatg gactaccaca tgtaaagact gatggacaaa ttgatatagt tagtgttgct ttaactattt atgattcaac aaccttgagg gataagattg aagaagttag aacgaatgca aacgatccta agtggacgga agaaagtcgt actgaggttt taacaggatt agatacaatt aagacagata ttgataataa tcctaagacg caaacagata ttgatagtaa aattgttgag gttaatgaat tagagaaatt gttagtattg tca | Nucleic acid sequence of SEQ ID NO: 6 |
| 6 | GPLGSASVLIGISFLGGFTQGQFNISTDTVFAAEVISGSAVTL NTNMTKNVQNGRAYIDLYDVKNGKIDPLQLITLNSPDLKAQY VIRQGGNYFTQPSELTTVGAASINYTVLKTDGSPHTKPDGQV DIINVSLTIYNSSALRDKIDEVKKKAEDPKWDEGSRDKVLISLD DIKTDIDNNPKTQSDIANKITEVTNLEKILVPRIPEFSTIPGSAA TLNTSITKNIQNGNAYIDLYDVKLGKIDPLQLIVLEQGFTAKYV FRQGTKYYGDVSQLQSTGRASLTYNIFGEDGLPHVKTDGQI DIVSVALTIYDSTTLRDKIEEVRTNANDPKWTEESRTEVLTGL DTIKTDIDNNPKTQTDIDSKIVEVNELEKLLVLS | Fusion protein of Rib-AlpC N-terminal regions |
| 7 | atggccgaag ttattagcgg tagcgcagca accctgaata gcgcactggt taaaaatgtt agcggtggca aagcctatat cgacatctat gatgtgaaaa acggcaaaat tgatccgctg aatctgattg ttctgcctcc gagcaattat agcgccaact attatatcaa acagggtggt cgcattttca ccaatgttaa tcagctgcag acaccgggta cagcaaccat tacctataac attctggatg aaaatggcaa cccgtatacc aaaagtgatg gccagattga tattgttagc ctggttacca ccgtttatga taccaccgaa ctgcgcaata acatcaacaa agttattgag aatgccaacg acccgaaatg gtcagatgat agccgtaaag | Nucleic acid sequence of SEQ ID NO: 8 |
| | atgttctgag caaaatcgag gtgatcaaaa acgatattga taacaacccg aaaacccaga gcgatatcga caacaaaatt gtggaagtga acgagctgga aaaactgctg gttctgccgt aataa | |
| 8 | MAEVISGSAATLNSALVKNVSGGKAYIDIYDVKNGKIDPLNLIV LTPSNYSANYYIKQGGRIFTSVNQLQTPGTATITYNILDENGN PYTKSDGQIDIVSLVTTVYDTTELRNNINKVIENANDPKWSDD SRKDVLSKIEVIKNDIDNNPKTQSDIDNKIVEVNELEKLLVLP | Alp1 N-terminal region |
| 9 | atggcaagca ccattccggg tagcgcagca accctgaata ccagcattac caaaaacatt cagaatggca acgcctatat cgatctgtat gatgtgaaaa acggtctgat tgatccgcag aatctgattg ttctgaatcc gagcagctat agcgccaact attatatcaa acagggtgcc aaatattaca gcaacccgag cgaaattacc accaccggta gcgcaaccat tacctttaac attctggatg aaaccggcaa cccgcataaa aaagcagatg gtcagattga tattgtgagc gttaacctga ccatttatga tagcaccgca ctgcgtaatc gtattgatga agttattaac aatgccaacg acccgaaatg gtctgatggt agccgtgatg aagtgctgac cggtctggaa aaaatcaaaa aagatatcga taacaacccg aaaacccaga tcgacattga caacaaaatt aacgaagtga acgaaatcga aaaactgctg gttgttagcc tgtaataa | Nucleic acid sequence of SEQ ID NO: 10 |
| 10 | ASTIPGSAATLNTSITKNIQNGNAYIDLYDVKNGLIDPQNLIVLN PSSYSANYYIKQGAKYYSNPSEITTTGSATITFNILDETGNPH KKADGQIDIVSVNLTIYDSTALRNRIDEVINNANDPKWSDGSR DEVLTGLEKIKKDIDNNPKTQIDIDNKINEVNEIEKLLVVSL | Alp2/Alp3 N-terminal region |
| 11 | atggccgaag ttattagcgg tagcgcagca accctgaata gcgcactggt taaaaatgtt agcggtggca aagcctatat cgacatctat gatgtgaaaa acggcaaaat tgatccgctg aatctgattg ttctgcctcc gagcaattat agcgccaact attatatcaa acagggtggt cgcattttca ccaatgttaa tcagctgcag acaccgggta cagcaaccat tacctataac attctggatg aaaatggcaa cccgtatacc aaaagtgatg gccagattga tattgttagc ctggttacca ccgtttatga taccaccgaa ctgcgcaata acatcaacaa agttattgag aatgccaacg acccgaaatg gtcagatgat agccgtaaag atgttctgag caaaatcgag gtgatcaaaa acgatattga taacaacccg aaaacccaga gcgatatcga caacaaaatt gtggaagtga acgagctgga aaaactgctg gttctgccgg aatttagcac cattccgggt tcagcagcca cactgaatac cagcattacc aaaaacattc agaatggcaa cgcctacatt gatctgtacg atgtaaaaaa tggtctgatc gatccgcaga acctgatcgt gctgaatccg agcagctatt cagccaatta ttatattaaa caaggcgcaa aatactatag caacccgagc gaaattacca ccaccggtag cgccaccatt acgtttaata tcctggacga aaccggtaac ccgcataaaa aagcagatgg tcaaattgat atcgtgagcg ttaacctgac gatttatgat agcacagccc tgcgtaatcg tattgatgaa gtgattaata acgcgaatga tcctaaatgg tccgatggta gtcgtgatga agtactgacc ggtctggaaa aaatcaaaaa agacatcgac aataatccga aaacgcagat tgacattgac aataaaatca acgaggtgaa cgagatcgag aaactgctgg tagttagcct gtaataa | Nucleic acid sequence of SEQ ID NO: 12 |
| 12 | MAEVISGSAATLNSALVKNVSGGKAYIDIYDVKNGKIDPLNLIV LTPSNYSANYYIKQGGRIFTSVNQLQTPGTATITYNILDENGN PYTKSDGQIDIVSLVTTVYDTTELRNNINKVIENANDPKWSDD SRKDVLSKIEVIKNDIDNNPKTQSDIDNKIVEVNELEKLLVLPE FSTIPGSAATLNTSITKNIQNGNAYIDLYDVKNGLIDPQNLIVLN | Fusion protein of Alp1-Alp2/Alp3 N-terminal |
| | PSSYSANYYIKQGAKYYSNPSEITTTGSATITFNILDETGNPH KKADGQIDIVSVNLTIYDSTALRNRIDEVINNANDPKWSDGSR DEVLTGLEKIKKDIDNNPKTQIDIDNKINEVNEIEKLLVVSL | regions |
| 13 | gtagttgaag gaagtgctgc aacattaaat actgccatga ctaaaaacat gcagaatggg aatgcatata ttgatattta tgatgttaaa ttaggaaaaa ttgatccatt acaactgatt aaattggaac cgggatacac tgctatttat tacattacac aaggttcaaa agtttatgca aatgtttcgg agctacaaac accaggagca gcgaaagtta attatcgtat tcaaacctct gatggaagcg atcatataaa atctgatggt caattagaca gcgttaatat ttcattaaca gtttatgatt | Nucleic acid sequence of SEQ ID NO: 14 |
| 14 | VVEGSAATLNTAMTKNMQNGNAYIDIYDVKLGKIDPLQLIKLE PGYTAIYYITQGSKVYANVSELQTPGAAKVNYRIQTSDGSDHI KSDGQLDSVNISLTVYD | N-terminal region of Alp4 |
| 15 | atggctgaag taatttcagg aagtgctgtt acgttaaaca caaatatgac taaaaatgtt cagaatggta gagcatatat agatttatat gatgtgaaaa atgggaaaat agatccatta caattaatta cgttaaattc acctgattta aaagctcagt atgtcattag gcaaggcggc aattatttca cacaaccttc tgaattgact actgttggtg cagctagtat taattataca gtattgaaga cagatggaag tcctcatacg aagcctgatg gacaagtgga tattataaac gtttcattga ctatttacaa ttcttcagct ttgagagata aaatagatga agttaaaaag aaagcggaag accctaaatg ggacgaggga agtcgcgata aagttttgat aagtttagat gatatcaaaa cagatattga taataatcct aagacgcaat cagacattgc caataaaata actgaagtta ctaatttaga aaaaatacta gtacctcgaa tccca | Nucleic acid sequence of SEQ ID NO: 16 |
| 16 | MAEVISGSAVTLNTNMTKNVQNGRAYIDLYDVKNGKIDPLQLI TLNSPDLKAQYVIRQGGNYFTQPSELTTVGAASINYTVLKTD GSPHTKPDGQVDIINVSLTIYNSSALRDKIDEVKKKAEDPKWD EGSRDKVLISLDDIKTDIDNNPKTQSDIANKITEVTNLEKILVPR IP | Shorter Rib N-terminal region |
| 17 | tctacaattc cagggagtgc agcgacctta aatacaagca tcactaaaaa tatacaaaac ggaaatgctt acatagattt atatgatgta aaattaggta aaatagatcc attacaatta attgttttag aacaaggttt tacagcaaag tatgttttta gacaaggtac taaatactat ggggatgttt ctcagttgca gagtacagga agggctagtc ttacctataa tatatttggt gaagatggac taccacatgt aaagactgat ggacaaattg atatagttag tgttgcttta actatttatg attcaacaac cttgagggat aagattgaag aagttagaac gaatgcaaac gatcctaagt ggacggaaga aagtcgtact gaggttttaa caggattaga tacaattaag acagatattg ataataatcc taagacgcaa acagatattg atagtaaaat tgttgaggtt aatgaattag agaaattgtt agtattgtca taataa | Nucleic acid sequence of SEQ ID NO: 18 |
| 18 | STIPGSAATLNTSITKNIQNGNAYIDLYDVKLGKIDPLQLIVLEQ GFTAKYVFRQGTKYYGDVSQLQSTGRASLTYNIFGEDGLPH VKTDGQIDIVSVALTIYDSTTLRDKIEEVRTNANDPKWTEESR TEVLTGLDTIKTDIDNNPKTQTDIDSKIVEVNELEKLLVLS | Shorter AlpC N-terminal region |
| 19 | agcaccattc cgggttcagc agccacactg aataccagca ttaccaaaaa cattcagaat ggcaacgcct acattgatct gtacgatgta aaaaatggtc tgatcgatcc gcagaacctg atcgtgctga atccgagcag ctattcagcc aattattata ttaaacaagg cgcaaaatac tatagcaacc cgagcgaaat | Nucleic acid sequence of SEQ ID NO: 20 |
| | taccaccacc ggtagcgcca ccattacgtt taatatcctg gacgaaaccg gtaacccgca taaaaaagca gatggtcaaa ttgatatcgt gagcgttaac ctgacgattt atgatagcac agccctgcgt aatcgtattg atgaagtgat taataacgcg aatgatccta aatggtccga tggtagtcgt gatgaagtac tgaccggtct ggaaaaaatc aaaaaagaca tcgacaataa tccgaaaacg cagattgaca ttgacaataa aatcaacgag gtgaacgaga tcgagaaact gctggtagtt agcctgtaat aa | |
| 20 | STIPGSAATLNTSITKNIQNGNAYIDLYDVKNGLIDPQNLIVLNP SSYSANYYIKQGAKYYSNPSEITTTGSATITFNILDETGNPHK KADGQIDIVSVNLTIYDSTALRNRIDEVINNANDPKWSDGSRD EVLTGLEKIKKDIDNNPKTQIDIDNKINEVNEIEKLLVVSL | Shorter Alp2/Alp3 N-terminal region |
| 21 | atggctgaagtaatttcaggaagtgctgttacgttaaacacaaatatgactaaaaatgtt cagaatggtagagcatatatagatttatatgatgtgaaaaatgggaaaatagatccatt acaattaattacgttaaattcacctgatttaaaagctcagtatgtcattaggcaaggcgg caattatttcacacaaccttctgaattgactactgttggtgcagctagtattaattatacagt attgaagacagatggaagtcctcatacgaagcctgatggacaagtggatattataaa cgtttcattgactatttacaattcttcagctttgagagataaaatagatgaagttaaaaag aaagcggaagaccctaaatgggacgagggaagtcgcgataaagttttgataagttta gatgatatcaaaacagatattgataataatcctaagacgcaatcagacattgccaata aaataactgaagttactaatttagaaaaaatactagtacctcgaatcccagaattctct acaattccagggagtgcagcgaccttaaatacaagcatcactaaaaatatacaaaa cggaaatgcttacatagatttatatgatgtaaaattaggtaaaatagatccattacaatt aattgttttagaacaaggttttacagcaaagtatgtttttagacaaggtactaaatactat ggggatgtttctcagttgcagagtacaggaagggctagtcttacctataatatatttggtg aagatggactaccacatgtaaagactgatggacaaattgatatagttagtgttgctttaa ctatttatgattcaacaaccttgagggataagattgaagaagttagaacgaatgcaaa cgatcctaagtggacggaagaaagtcgtactgaggttttaacaggattagatacaatt aagacagatattgataataatcctaagacgcaaacagatattgatagtaaaattgttg aggttaatgaattagagaaattgttagtattgtcataataa | Nucleic acid sequence of SEQ ID NO: 22 |
| 22 | MAEVISGSAVTLNTNMTKNVQNGRAYIDLYDVKNGKIDPLQLI TLNSPDLKAQYVIRQGGNYFTQPSELTTVGAASINYTVLKTD GSPHTKPDGQVDIINVSLTIYNSSALRDKIDEVKKKAEDPKWD EGSRDKVLISLDDIKTDIDNNPKTQSDIANKITEVTNLEKILVPR IPEFSTIPGSAATLNTSITKNIQNGNAYIDLYDVKLGKIDPLQLI VLEQGFTAKYVFRQGTKYYGDVSQLQSTGRASLTYNIFGED GLPHVKTDGQIDIVSVALTIYDSTTLRDKIEEVRTNANDPKWT EESRTEVLTGLDTIKTDIDNNPKTQTDIDSKIVEVNELEKLLVL S | Fusion protein of shorter Rib-AlpC N-terminal regions |

## Claims

1. An immunogenic complex comprising
i) an amino acid sequence of an N-terminal region of a group B Streptococcus surface protein selected from the group consisting of:
a first amino acid sequence according to SEQ ID NO: 16, or an amino acid sequence having at least 98%, such as 99%, identity thereto;
a second amino acid sequence according to SEQ ID NO: 18 or an amino acid sequence having at least 98%, such as 99%, identity thereto; and
a third amino acid sequence according to SEQ ID NO: 20; and
ii) a capsular polysaccharide.

2. The immunogenic complex according to claim 1, wherein said first amino acid sequence consists of SEQ ID NO: 16.

3. The immunogenic complex according to claim 1, wherein said second amino acid sequence consists of SEQ ID NO: 18.

4. The immunogenic complex according to claim 1, wherein said third amino acid sequence consists of SEQ ID NO: 20.

5. The immunogenic complex according to any one of the preceding claims, wherein said amino acid sequence of an N-terminal region of a group B Streptococcus surface protein of said immunogenic complex is either said first amino acid sequence, said second or said third amino acid sequence.

6. The immunogenic complex according to any one of the preceding claims, wherein said immunogenic complex comprises two or more of said first amino acid sequence, said second amino acid sequence and said third amino acid sequence.

7. The immunogenic complex according to any of the preceding claims, further comprising a further amino acid sequence having at least 80% sequence identity with the amino acid sequence of the N-terminal region of a further group B Streptococcus surface protein, such as a group B Streptococcus surface protein selected from the group consisting of Rib protein, Alp1 protein, Alp2 protein, Alp3 protein, Alp 4 protein and AlpC protein, such as the group consisting of Alp1 protein, Alp2 protein, Alp3 protein, and Alp 4 protein.

8. The immunogenic complex according to claim 7, wherein the further group B Streptococcus surface protein is not Rib when said immunogenic complex comprises said first amino acid sequence, is not AlpC when said immunogenic complex comprises said second amino acid sequence and/or is not Alp2 when said immunogenic complex comprises said third amino acid sequence.

9. The immunogenic complex according to any of the preceding claims, wherein said first, second and/or third amino acid sequence and said capsular polysaccharide, and optionally also the further group B Streptococcus surface protein, are derived from different group B Streptococcus serotypes.

10. The immunogenic complex according to any one of the preceding claims, wherein said first amino acid sequence, said second amino acid sequence and/or said third amino acid sequence is/are conjugated to the capsular polysaccharide, such as by reductive amination, via a linker, via biotin-biotin-binding moiety, and/or sialic acid-sialic acid binding moiety.

11. The immunogenic complex according to any of preceding claims, wherein said first amino acid sequence, said second amino acid sequence and/or said third amino acid sequence is/are modified by glycosylation, amidation, carboxylation or phosphorylation, or by being conjugated to an RSV antigen.

12. An immunogenic product comprising the immunogenic complex according to any one of the preceding claims, wherein the immunogenic product further comprises an immunogenic fusion protein comprising:
a first amino acid sequence having at least 80% sequence identity with the amino acid sequence of the N-terminal region of a first group B Streptococcus surface protein, which is fused to
a second amino acid sequence having at least 80% sequence identity with the amino acid sequence of the N-terminal region of a second group B Streptococcus surface protein,
wherein each of the first and the second group B Streptococcus surface protein is selected from the group consisting of Rib protein, Alp1 protein, Alp2 protein, Alp3 protein, Alp4 protein and AlpC protein, and wherein the immunogenic fusion protein is capable of eliciting protective immunity against group B Streptococcus.

13. The immunogenic product according to claim 12, wherein the first amino acid sequence of said fusion protein has at least 80%, such as at least 85%, such as at least 90%, such as 95, 96, 97, 98 or 99 % sequence identity with one of the amino acid sequences SEQ IDs 2, 4, 8, 10, 14, 16, 18 and 20, and wherein the second amino acid sequence of said fusion protein has at least 80%, such as at least 85%, such as at least 90%, such as 95, 96, 97, 98 or 99 % sequence identity with one of the amino acid sequences SEQ IDs 2, 4, 8, 10, 14, 16, 18 and 20,
or alternatively,
wherein the immunogenic fusion protein comprises an amino acid sequence having at least 80%, such as at least 85%, such as at least 90%, such as 95, 96, 97, 98 or 99 % sequence identity with any one of the amino acid sequences SEQ ID NO: 6, SEQ ID NO: 12, and SEQ ID NO: 22.

14. A vaccine comprising or consisting of a pharmaceutically acceptable vehicle, optionally an adjuvant, such as aluminium hydroxide, and a pharmaceutically effective amount of an immunogenic complex according to any one of claims 1-11 and/or a pharmaceutically effective amount of an immunogenic product according to claim 12 or 13.

15. The immunogenic complex according to any of claims 1-11, the immunogenic product according to claim 12 or 13, and/or the vaccine according to claim 14 for use in a method of preventing or treating an infection caused by a group B Streptococcus.
